(19) Europäisches Patentamt
European Patent Office
Office européen des brevets

(11) **EP 1 057 524 B1**

(12) **EUROPÄISCHE PATENTSCHRIFT**

(45) Veröffentlichungstag und Bekanntmachung des Hinweises auf die Patenterteilung:
**05.03.2008 Patentblatt 2008/10**

(51) Int Cl.:
***B01J 8/06*** *(2006.01)*    ***C07C 45/50*** *(2006.01)*

(21) Anmeldenummer: **00108156.1**

(22) Anmeldetag: **13.04.2000**

(54) **Verfahren zur katalytischen Durchführung von Hydroformylierungen**

Process to perform catalysed hydroformylations

Procédé de réalisation des réactions catalysées d'hydroformylations

(84) Benannte Vertragsstaaten:
**AT BE CH CY DE DK ES FI FR GB GR IE IT LI LU MC NL PT SE**

(30) Priorität: **02.06.1999 DE 19925384**

(43) Veröffentlichungstag der Anmeldung:
**06.12.2000 Patentblatt 2000/49**

(73) Patentinhaber: **Evonik Oxeno GmbH**
**45772 Marl (DE)**

(72) Erfinder:
• **Wiese, Klaus-Diether, Dr.**
**45721 Haltern (DE)**
• **Protzmann, Guido, Dr.**
**45772 Marl (DE)**
• **Koch, Jürgen**
**45721 Haltern (DE)**
• **Röttger, Dirk, Dr.**
**45657 Recklinghausen (DE)**
• **Trocha, Martin, Dr.**
**45136 Essen (DE)**

(74) Vertreter: **Hirsch, Hans-Ludwig et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
EP-A- 0 987 242          DE-A- 2 538 037
DE-A- 2 715 685          DE-A- 3 234 701
GB-A- 938 831            GB-A- 938 836

EP 1 057 524 B1

**Beschreibung**

Die vorliegende Erfindung betrifft ein Verfahren zur Durchführung von Mehrphasenreaktionen in einem Rohrreaktor, insbesondere zur Herstellung von Aldehyden durch Umsetzung von olefinisch ungesättigten Verbindungen mit Wasserstoff und Kohlenmonoxid in Anwesenheit eines Katalysators.

Aldehyde werden bei der Synthese vieler organischer Verbindungen eingesetzt. Ihre direkten Folgeprodukte sind Alkohole und Carbonsäuren, die technisch genutzt werden. Die aus den Aldehyden hergestellten Alkohole finden u.a. Verwendung als Lösungsmittel und als Vorstufe für die Herstellung von Weichmachern und Detergentien.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, hat in letzter Zeit Rhodium zunehmende Bedeutung erlangt. Im Gegensatz zu Kobalt gestattet Rhodium, die Reaktion bei niedrigerem Druck durchzuführen. Die Hydrierung der Olefine zu gesättigten Kohlenwasserstoffen ist bei Anwendung von Rhodium-Katalysatoren deutlich geringer als bei Anwendung von Kobalt-Katalysatoren.

Bei den in der Technik durchgeführten Verfahren wird der Rhodium-Katalysator während des Prozesses aus einem Katalysatorvorläufer, Synthesegas und gegebenenfalls weiteren modifizierend wirkenden Liganden gebildet. Bei Einsatz von modifizierten Katalysatoren können die modifizierenden Liganden im Überschuss im Reaktionsgemisch vorliegen. Besonders bewährt haben sich als Liganden tertiäre Phosphine oder Phosphite. Ihre Anwendung ermöglicht, den Reaktionsdruck auf Werte unter 300 bar zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Reaktionsprodukt aus dem Reaktionsgemisch ab. In der Praxis kann dieser Weg wegen der thermischen Empfindlichkeit des Katalysators oder der gebildeten Produkte nur bei der Hydroformylierung niedriger Olefine mit bis zu etwa 5 Kohlenstoffatomen im Molekül beschritten werden.

Großtechnisch werden $C_4$- und $C_5$-Aldehyde beispielsweise nach DE 3234701 oder DE 2715685 hergestellt.

Im letztgenannten Verfahren ist der Katalysator in der organischen Phase gelöst, die aus Produkt und Hochsieder (entstanden aus dem Produkt) besteht. In dieses Gemisch wird Olefin und Synthesegas eingeleitet. Das Produkt wird mit dem Synthesegas aus dem Reaktor ausgetragen, in einer neueren Variante als Flüssigkeit abgezogen. Da der Katalysator langsam in seiner Leistung nachläßt, muß ständig ein Teil zusammen mit Hochsieder ausgeschleust und durch eine äquivalente Menge ersetzt werden. Wegen des hohen Rhodiumpreises ist die Rückgewinnung des Rhodiums aus dem Ausschleusestrom unerläßlich. Der Aufarbeitungsprozeß ist komplex und belastet somit das Verfahren.

Gemäß DE 3234701 wird dieser Nachteil beispielsweise dadurch überwunden, daß der Katalysator in Wasser gelöst ist. Die Wasserlöslichkeit des verwendeten Rhodiumkatalysators wird durch die Verwendung von trisulfonierten Triarylphosphinen als Liganden erreicht. In die wässerige Phase wird Olefin und Synthesegas eingeleitet. Das durch die Reaktion entstehende Produkt bildet eine zweite flüssige Phase. Die flüssigen Phasen werden außerhalb des Reaktors getrennt und die abgetrennte Katalysatorphase in den Reaktor zurückgeführt.

Technisch gesehen handelt es sich bei beiden genannten Prozessen um Mehrphasenreaktionen.

Unter Mehrphasenreaktionen werden im folgenden Reaktionen verstanden, die unter Beteiligung von zwei oder mehr nicht oder nur teilweise mischbaren fluiden Phasen ablaufen. Dies betrifft z.B. Reaktionen zwischen einer Gas- und einer Flüssigphase (gl), zwischen zwei Flüssigphasen, die nicht mischbar sind oder eine Mischungslücke aufweisen (ll) und Reaktionen, an denen sowohl zwei flüssige nicht oder nur teilweise mischbare Phasen sowie eine Gasphase beteiligt sind (gll).

Darüber hinaus ist aber auch der Einsatz anderer fluider Phasen, z.B. überkritischer Phasen denkbar. Eine solche überkritische Phase kann alternativ zur den genannten, aber auch zusätzlich auftreten.

Beispiele für technisch wichtige Gas-Flüssig-Reaktionen (gl) sind neben der Hydroformylierung von flüssigen Olefinen mit einem in organischer Phase gelöstem Katalysator die Reaktion von Acetylen mit Carbonsäuren oder Hydrierungen mit homogen gelösten Katalysatoren oder Oxidationen mit Luft oder Sauerstoff.

Die genannten Fälle haben das Problem des Stoffübergangs gemeinsam, da die Reaktionspartner in unterschiedlichen Phasen vorliegen. Im Falle der Hydroformylierung im 3-Phasensystem ist die Verfahrensführung besonders schwierig, da die Edukte in drei getrennten Phasen vorliegen. Sowohl Olefin als auch Synthesegas müssen in die wäßrige Katalysatorphase transportiert werden, um dort mit dem Katalysator in Kontakt zu kommen. Schließlich muß der Rücktransport aus der wäßrigen Phase erfolgen. Da die Transportvorgänge häufig langsamer sind als die eigentliche Reaktion, sind solche Reaktionen durch die Geschwindigkeit des Stoffüberganges bestimmt, man spricht von einer transportgehemmten Reaktion. Mehrphasenreaktionen sind mit einer Reihe von Problemen verbunden, die ihre technische Ausführung wesentlich schwieriger machen als bei einfachen homogenen Reaktionen. Im Folgenden sind einige typische Probleme erwähnt:

In allen Fällen müssen die Stoffe möglichst innig miteinander in Kontakt gebracht werden, um das Problem des Stoffübergangs zu minimieren: es muß eine möglichst große Stoffübergangsfläche $a_s$ zwischen den Phasen erzeugt werden. Andererseits müssen die Phasen nach erfolgter Reaktion wieder leicht getrennt werden können: Zu starke Vermischung kann hier zu Problemen führen. Bei Anwesenheit zweier flüssiger Phasen kann es zur Emulsionsbildung kommen, bei Gas-Flüssig-Verfahren zum Schäumen. Bei dem erwähnten 3-Phasenverfahren können sogar alle Probleme gleichzeitig auftreten.

Neben einer hohen Stoffübergangsfläche $a_s$ sollte in allen Mehrphasenreaktionen ein möglichst hoher Stoffübergangskoeffizient $k_l$ erreicht werden. Insgesamt sollte der sogenannte KLA-Wert, d.h. das Produkt aus $k_l$ und $a_s$ in der Stoffübergangsgleichung

$$j = k_l * a_s * (C^* - C)$$

mit

j      [Mol/s] der durch die Phasengrenzfläche hindurchtretende Molenstrom der reagierenden Komponente,
$k_l$      [m/s] Stoffübergangskoeffizient,
$a_s$      [$m^2$] Phasengrenzfläche im Reaktor,
$C^*$      [Mol/$m^3$] maximale Löslichkeit des Edukts in der zweiten Phase und
C      [Mol/$m^3$] tatsächliche Konzentration des Edukts, die wiederum mit der Reaktionsgeschwindigkeit gekoppelt ist,

maximal sein.

Ein weiteres Problem bei Mehrphasenreaktionen ist die Wärmeabfuhr bei exothermen Reaktionen. Gelingt es, die Reaktionsgeschwindigkeit durch Verbesserung des Stoffüberganges zu erhöhen, muß naturgemäß auch mehr Wärme abgeführt werden, was zu unerwünschter Temperaturerhöhung bis hin zum Durchgehen der Reaktion führen kann.

Diese Problematik der Mehrphasenreaktion kann technisch z.B. durch die Verwendung eines Rührkesselreaktors gelöst werden.

Für die Hydroformylierung von Olefinen ist die Verwendung eines Rührwerks unter erhöhtem Druck nachteilig, da hierbei eine störanfällige Wellenabdichtung vorzusehen ist. Des weiteren müssen in diesem Verfahren mehrere Rührer zum Einsatz kommen, um eine hinreichende Phasenvermischung zu erzielen.

Die bei Hydroformylierungen anfallende hohe Reaktionswärme kann in der Regel nur durch Verwendung von im Inneren des Reaktors angebrachten Wärmetauschern kontrolliert werden.

Die Verwendung eines Rührkessels führt immer zu einer Rückvermischung, wodurch die effektive Konzentration der Reaktanden herabgesetzt wird, was zur Absenkung der Raumzeitausbeute führt. Dieser Nachteil muß mit der Investition von teurem Reaktionsraum bezahlt werden.

Bei der Hydroformylierung von Olefinen nimmt die Raum-Zeit-Ausbeute mit steigender Zahl der Kohlenstoffatome im Olefin ohnehin stark ab. Der Zusammenhang zwischen Molekülgröße und Reaktionsgeschwindigkeit ist bekannt (B.Cornils, W.Herrmann, Aqueous-Phase Organometallic Catalysis, Concepts and Application, Verlag Wiley-VCH, S. 308-310).

So liegt zum Beispiel das Verhältnis der Reaktionsgeschwindigkeiten der Hydroformylierung von 1-Penten zu 1-Hexen bei 2,6/l. Eine Umsetzung von höheren Olefinen wird daher immer unwirtschaftlicher.

Im Hinblick auf die voranstehenden Darlegungen besteht ein Bedarf für ein Verfahren, das die vorstehend genannten Nachteile vermeidet und sich zudem auf einfache Weise technisch realisieren läßt.

Aufgabe der vorliegenden Erfindung ist somit die Entwicklung eines Verfahrens zur Durchführung von Mehrphasenreaktionen, das sich besonders für die Herstellung von Aldehyden mittels katalytischer Hydroformylierung von Olefinen eignet.

Technisch sollte das neue Verfahren folgende Ansprüche an ein Mehrphasenverfahren erfüllen:

• Erzeugung eines hohen und stabilen Stoffübergangs zwischen den beteiligten Phasen

• Einfach ausführbar, möglichst mit üblichen technischen Apparaten

• Einfache und sichere Wärmeabfuhr

• Hohe Betriebssicherheit

• Einfache und sichere Maßstabsübertragung

In Bezug auf die durchzuführende Herstellung von Aldehyden kommen speziell hinzu:

3

- Hohe Selektivität, Vermeidung insbesondere hochsiedender Nebenprodukte
- Keine oder nur geringfügige Katalysatorausschleusung
- Hohe Raum-Zeit-Ausbeute, kleine Reaktoren
- Hohe Produktreinheit

Mit dem erfindungsgemäßen Verfahren wurde ein überraschend einfaches Verfahren zur Durchführung von Mehrphasenreaktionen gefunden, das in einem Rohrreaktorgegebenenfalls gefüllt mit Füllkörpern oder Einbauten - durchgeführt werden kann und zur Hydroformylierung von Olefinen mit Synthesegas unter hohen Raum-Zeit-Ausbeuten und Selektivitäten geeignet ist.

Gegenstand der vorliegenden Erfindung ist daher ein Verfahren zur katalytischen Durchführung von Mehrphasenreaktionen in einem Rohrreaktor, wobei der Katalysator in der kontinuierlichen Phase und mindestens ein Edukt in einer dispergierten Phase enthalten ist und der Belastungsfaktor B des Reaktors gleich oder größer 0,8 ist und durch die Mehrphasenreaktion Olefine hydroformyliert, d. h. mit Synthesegas zu Aldehyden umgesetzt werden.

Der im erfindungsgemäßen Verfahren eingesetzte Rohrreaktor kann Füllkörper oder Einbauten enthalten. Füllkörper im Sinne der vorliegenden Erfindung sind beispielsweise: Raschigringe, Sättel, Pallringe, Telleretten, Maschendrahtringe, Maschendrahtgewebe. Beispiele für Einbauten sind Filterplatten, Strombrecher, Kolonnenböden, Lochbleche oder sonstige Mischvorrichtungen. Als Einbauten im Sinne der vorliegenden Erfindung sind aber auch mehrere enge, parallel geschaltete Rohre denkbar, es resultiert also ein Viellrohrreaktor. Besonders bevorzugt sind strukturierte Mischerpackungen oder Demisterpackungen.

Entscheidende Bedeutung hat im erfindungsgemäßen Verfahren die Einhaltung bzw. Überschreitung einer minimalen Querschnittsbelastung des Rohrreaktors. Bei Aufwärtsbetrieb des Reaktors (Strömungsrichtung von unten nach oben) sollte der Flutpunkt überschritten werden. Der Reaktor wird also oberhalb des Punktes betrieben, bei dem man üblicherweise Blasensäulen betreibt. Bei Abwärtsbetrieb (Strömungsrichtung von oben nach unten) ist die Querschnittsbelastung so einzustellen, daß der Reaktor vollständig geflutet ist. Man arbeitet somit oberhalb des Punktes, bei dem man noch von einer Rieselphase (trickle bed) sprechen kann.

Zur genaueren Festlegung der minimal einzuhaltenden Belastung des Reaktors wird der Belastungsfaktor B des Rohrreaktors als ein dimensionsloser Druckverlust mit

$$B = PD/PS$$

berechnet, wobei PD [Pa/m] ein längenbezogener Druckverlust über den Reaktor unter Betriebsbedingungen und PS [Pa/m] eine Rechengröße mit der Dimension eines längenbezogenen Druckes, definiert als Verhältnis von Massenstrom M [kg/s] aller Komponenten im Reaktor zum Volumenstrom V [m$^3$/s] aller Komponenten unter Betriebsbedingungen, multipliziert mit g = 9,81 m/s$^2$, d. h. PS = (M/V)*g, bedeuten.

Anschaulich wäre PS der statische Druck pro Meter eines Mehrphasengemisches in einem senkrecht stehenden Rohr, wenn alle Phasen mit gleicher Geschwindigkeit strömen würden. PS ist eine reine Rechengröße, die sich aus den dem Reaktor zugeführten Mengenströmen ergibt und die unabhängig von der Strömungsrichtung des Reaktors, der Strömungsgeschwindigkeit aller Phasen oder des Flutzustandes des Reaktors angegeben werden kann.

Der Druckverlust PD [Pa/m] wird als Rechengröße, um die Prozeßbedingungen festzulegen, verwendet und kann nach den gängigen Methoden für Ein- bzw. Mehrphasenströmungen berechnet werden. Gängige Verfahren zur Berechnung des Druckverlustes PD in Rohren, Einbauten oder Füllkörperschüttungen usw. können z. B. im VDI-Wärmeatlas 7. Erweiterte Auflage, VDI-Verlag GmbH, Düsseldorf 1994, Abschnitte La1 bis Lgb7, sowie im Standardwerk Heinz Brauer, Grundlagen der Einphasen- und Mehrphasenströmungen, Verlag Sauerländer, Aarau und Frankfurt am Main, 1971, nachgeschlagen werden.

Der Druckverlust PD ist bei der Einphasenströmung durch ein leeres Rohr durch

$$PD = Cw*\rho/2*w^2/D$$

mit

$\rho$     [kg/m$^3$] Dichte des strömenden Mediums unter Betriebsbedingungen,
w     [m/s] Strömungsgeschwindigkeit (Volumenstrom/Querschnittsfläche),
D     [m] Rohrdurchmesser und

Cw   [-] Widerstandsbeiwert des durchströmten Rohres

gegeben.

Bei einer Strömung durch Füllkörper, Schüttungen oder Einbauten ist die Geschwindigkeit w durch die Effektivgeschwindigkeit (w/ψ) sowie der Rohrdurchmesser D durch den hydraulischen Kanaldurchmesser $d_H$ der Füllkörper oder Einbauten zu ersetzen, so daß gilt:

$$PD = Cw * \rho/2 * (w/\psi)^2 * 1/d_H$$

mit

$d_H$   [m] Hydraulischer Kanaldurchmesser
ψ   [-] Leerrohranteil
Cw   [-] Widerstandsbeiwert des durchströmten Apparates mit Füllung

Die füllkörperspezifischen Daten $d_H$ und ψ sind häufig Bestandteil der Lieferspezifikationen von Füllkörpern. Für eine Reihe von Füllkörpern sind Daten im oben erwähnten VDI-Wärmeatlas angegeben.

Der Leerrohranteil ψ kann auch experimentell bestimmt werden, indem man zum Beispiel den Reaktor vor und nach Befüllung mit den Füllkörpern auslitert. Der hydraulische Kanaldurchmesser wiederum kann, wenn er nicht bekannt ist, aus der spezifischen Oberfläche F $[m^2/m^3]$ der Füllkörper oder Einbauten (in der Regel bekannt oder experimentell bestimmbar) nach der einfachen Beziehung

$$d_H = 4\psi/F.$$

berechnet werden.

Der Widerstandsbeiwert von Rohren, Einbauten und Füllkörpern wird in der Regel in Abhängigkeit von der Reynoldszahl Re beschrieben, die Auskunft über den Strömungszustand unter den gewählten Bedingungen gibt. Bei Füllkörpern, Einbauten usw. ist fast immer folgende Beziehung anwendbar:

$$Cw = K_1/Re^n + K_2/Re^m$$

wobei häufig n = 1, m = 0 (Ansatz nach S. Ergun, Chem. Engng. Progr. 48, (1948), 89), oder n = 1, m = 0,1 (Ansatz nach Brauer et al.) verwendet wird. $K_1$, $K_2$ sind fullkörperspezifische Konstanten, die aus Lieferdaten oder aus der Literatur bekannt sind (Beispiele sind im VDI-Wärmeatlas und bei Brauer et al. zu finden). Sie können aber auch experimentell bestimmt werden, indem man den Rohrreaktor mit Füllkörpern mit einer Flüssigkeit unter verschiedenen Geschwindigkeiten betreibt und aus den bekannten Daten und dem gemessenen Druckverlust Cw in Abhängigkeit von Re bestimmt.

Die dimensionslose Reynoldszahl Re schließlich ist definiert als

$$Re = w * (\rho/\eta) * D \text{ für Leerrohre bzw}.$$

Re = (w/ψ) * (ρ/η)*$d_H$ für Rohre mit Einbauten oder Füllkörpern. η [Pa*s] bezeichnet jeweils die Viskosität und ρ $[kg/m^3]$ die Dichte des strömenden Mediums.

Der Druckverlust bei Zweiphasenströmungen (hier gas-flüssig für Synthesegas/Katalysatorlösung) steigt überproportional an. Meist wird nach Lockhart-Martinelli (in Brauer et al.) der Druckverlust der Zweiphasenströmung $P_{lg}$ auf den Druckverlust einer der beiden Phasen bezogen, zum Beispiel auf den Druckverlust der reinen strömenden flüssigen Phase $P_l$, und in Beziehung zu dem Verhältnis des Druckverlustes der beiden als allein strömend gedachten Phasen $P_l$ und $P_g$ gesetzt.

Zur Berechnung von Druckverlusten in Zweiphasenströmungen werden häufig dimensionslose Drücke nach $\phi^2 = P_{lg}/P_l$ und $X^2 = P_l/P_g$ eingesetzt. Der weitere Zusammenhang $\phi^2 = \text{Funktion}(X^2)$ ist vielfach untersucht. Beispiele finden sich in folgenden Literaturstellen:

Y. Sato, T.Hirose, F. Takahashi, M. Toda: "Pressure Loss and Liquid Hold Up in Packed Bed Reactor with Cocurrent Gas-Liquid Down Flow"; J. Chem. Eng. Of Japan, Vol 6 (Nr. 2), 1973, 147-152;

D. Sweeney: "A Correlation for Pressure Drop in Two-Phase Concurrent Flow in Packed Beds"; AIChE-Journal, Vol.13, 7/1967, 663-669;

V. W. Weekman, J. E. Myers: "Fluid-Flow Characteristics of Concurrent Gas-Liquid Flow in Packed Beds"; AIChE-Journal, Vol 10 (Nr. 6), 11/1964, 951-957;

R. P. Larkins, R. P. White, D. W. Jeffrey: "Two-Phase Concurrent Flow in Packed Beds"; AIChE-Journal, Vol 7 (Nr. 2), 6/1961, 231-239 oder

N. Midoux, M. Favier, J.- C. Charpentier: " Flow Pattern, Pressure Loss and Liquid Holdup Data in Gas- Liquid Down-Flow Packed Beds with Foaming and Nonfoaming Liquids"; J. Chem. Eng. Of Japan, Vol 9 (Nr. 5), 1976, 350-356.

Häufig wird für die Berechnung der von Midoux vorgeschlagene Zusammenhang, der für viele Gas-Flüssig-Systeme überprüft wurde, benutzt. Bei nichtschäumenden Systemen ist beispielsweise

$$\phi^2 = 1 + 1/X + 1,14/X^{0,54}$$

Dieser nach Lockart-Martinelli benannte Zusammenhang ist in vielen Werken graphisch dargestellt, detaillierte Abhandlungen hierüber finden sich in vielen Lehrbüchern der Verfahrenstechnik und Veröffentlichungen, so auch bei Brauer et al.

Der Druckverlust der Zweiphasenströmung $P_{gl}$ ergibt sich aus dem experimentell bestimmten oder wie oben erläutert abgeschätzten Druckverlust der reinen strömenden Flüssigphase $P_l$ dann mit

$$P_{gl} = \phi^2 * P_l$$

Im speziellen Fall der Herstellung von Aldehyden durch Hydroformylierung von Olefinen gestaltet sich die Berechung des Druckverlustes noch komplexer. Es ist neben der Synthesegas- und einer flüssigen Katalysatorphase die Gegenwart einer organischen Flüssigphase zu berücksichtigen. Dieser Problematik kann durch die Bestimmung eines weiteren dimensionslosen Druckes $\varphi^2_{org} = P_{gll}/P_{lg}$ Rechnung getragen werden, so daß der Druckverlust wie folgt zu bestimmen ist:

$$P_{gll} = \phi^2 * \phi^2_{org} * P_l$$

Allgemein gilt mit der Reaktorlänge L [m]

$$PD = P_{gl}/L \quad bzw. \ PD = P_{gll}/L$$

Der Druckverlust einer Mehrphasenströmung ist somit durch übliche Mittel der chemischen Verfahrenstechnik berechenbar. Analoges gilt für den zuvor definierten dimensionslosen Druckverlust B, d. h. den Belastungsfaktor des Mehrphasenreaktors.

Die Größe des dimensionslosen Belastungsfaktors B stellt eine notwendige Grundbedingung des erfindungsgemäßen Verfahrens dar; B sollte größer oder gleich 0.8, bevorzugt größer oder gleich 0,9 oder besonders bevorzugt größer oder gleich 1 sein.

Im Bereich B größer oder gleich 0,8 beginnt ein von oben nach unten betriebener Reaktor zu fluten. Es sei ausdrücklich darauf hingewiesen, daß bei Einhaltung dieser Bedingungen die Vorteile des erfindungsgemäßen Verfahrens auch dann erzielt werden, wenn der Reaktor von unten nach oben oder in anderer Richtung betrieben wird.

Höhere Querschnittsbelastungen des Reaktors (B >> 1), erkennbar am steigenden Differenzdruck über den Reaktor, sind jederzeit möglich und sogar erwünscht, solange die steigenden Raum-Zeit-Ausbeuten den gleichermaßen steigenden Energieverbrauch rechtfertigen. Eine Obergrenze ist daher nur durch praktische Überlegungen wie Energieverbrauch oder Schwierigkeiten bei der Trennung der Phasen nach erfolgter Reaktion gegeben.

Es ist somit ersichtlich, daß neben den Volumenströmen der einzelnen Phasen bzw. den hieraus abgeleiteten Leerrohrgeschwindigkeiten w = V/(πD²/4) die Apparateabmessungen des Reaktors (Länge L, Durchmesser D) sowie insbesondere die Daten der verwendeten Füllkörper (hydraulischer Durchmesser $d_H$, Leerrohranteil Ψ) eine wichtige Rolle spielen. Über die richtige Wahl dieser Parameter kann das Verfahren unschwer an die unterschiedlichsten Erfordernisse angepaßt werden, wichtig ist nur die Einhaltung der Forderung B >= 0,8, bevorzugt B >= 0,9 und besonders bevorzugt B >= 1.

Bei einer langsamen Reaktion wird man beispielsweise den hydraulischen Durchmesser der Füllkörper klein bzw. ihre spezifische Oberfläche groß wählen, so daß die geforderten Bedingungen für B schon bei kleinen Strömungsgeschwindigkeiten erreicht werden. Man erhält auf diese Weise ausreichende Verweilzeiten über die Länge eines technisch vernünftig dimensionierten Reaktors. Bei sehr schnellen Reaktionen empfiehlt sich eine umgekehrte Verfahrensweise.

Ein weiteres Kriterium bei der Durchführung des erfindungsgemäßen Verfahrens ist das Verhältnis des Massenstroms der flüssigen, den Katalysator enthaltenden Phase $M_1$ zu dem Massenstrom der oder den dispersen Phasen $M_2$ Im Fall der Hydroformylierung ist der Massenstrom der Katalysatorphase $M_1$ wesentlich größer als der Massenstrom der dispersen Phasen, d. h. der organischen Olefinphase $M_{2a}$ und der Synthesegasphase $M_{2b}$. Im erfindungsgemäßen Verfahren kann das Massenverhältnis $M_1/M_2$ der kontinuierlichen Phase ($M_1$) zu den dispersen Phasen ($M_2$) größer 2 sein, vorzugsweise gilt $M_1/M_2 > 10$. Strömungsverhältnisse mit $M_1/M_2 > 100$ sind durchaus möglich und häufig sogar vorteilhaft. Unter der Bedingung $M_1/M_2 > 2$ ist die Katalysatorphase die kontinuierliche Phase, während die dispersen Phasen in feine Blasen bzw. in feine Tropfen zerteilt werden. Im erfindungsgemäßen Verfahren ist es möglich, daß mindestens ein Edukt durch die von der kontinuierlichen Phase in den Rohrreaktor eingebrachte Energie dispergiert wird. Dies führt zu einer Verteilung zumindest eines Edukts in Blasen oder Tropfen innerhalb der kontinuierlichen Katalysatorphase.

Auch dies läßt sich mit üblichen ingenieurtechnischen Mitteln abschätzen. Es eignen sich hierfür Ansätze mit dimensionslosen Kennzahlen wie

$$d_S/d_H = k * Re_{gl(gll)}{}^m * We_{gl(gll)}{}^n$$

mit

| | |
|---|---|
| $d_s$ | Durchmesser der Tropfen bzw Blasen nach Sauter (in Brauer et al.) |
| $d_H$ | hydraulischer Füllkörperdurchmesser, |
| $Re_{gl(gll)}$ | Reynoldszahl der Mehrphasenströmung = $W_{gl(gll)} * (\rho_l/\eta_l) * (d_H/\Psi)$, |
| $We_{gl(gll)}$ | Weberzahl der Mehrphasenströmung = $W_{gl(gll)}^2 * (\rho_l/\sigma_{gl}) * (d_H/\Psi)^2$, |
| $k, m, n$ | empirische Konstanten (bekannt oder durch Versuche zu ermitteln), |
| $w$ | Leerrohrgeschwindigkeiten [m/s] = $V/(\pi D^2/4)$, |
| $V$ | Volumenstrom unter Betriebsbedingungen [m³/s], |
| $\rho$ | Dichte unter Betriebsbedingungen [kg/m³], |
| $\eta$ | Viskosität unter Betriebsbedingungen [Pa*s] und |
| $\gamma$ | Grenzflächenspannung unter Betriebsbedingungen [N/m] |

und den Indices l (Flüssigphase), g (Gasphase), gl (Gas/Flüssig-Zweiphasenströmung) und gll (Gas/Flüssig/Flüssig-Dreiphasenströmung).

Bei strukturierten Packungen wie Sulzer-SMV oder engen Rohren als Einbauten scheint plausibel, daß ein berechneter Blasen- bzw. Tropfendurchmesser $d_s$ größer als der Kanaldurchmesser nicht sinnvoll ist. Dies gilt aber nicht für durchlässige Packungen und Füllkörper wie beispielsweise Maschendrahtringe oder Maschendrahtgewebe (sogenannte Demisterpackungen oder Tropfenabscheider). Im erfindungsgemäßen Verfahren können berechnete Tropfendurchmesser verwendet werden, die mindestens gleich oder kleiner als der hydraulische Kanaldurchmesser sind:

$$d_S/d_H <= 1, \text{ vorzugsweise} < 0,9.$$

Aus dem berechneten Tropfendurchmesser läßt sich schließlich eine Stoffübergangsfläche nach

$$A_S = 6\varphi_g d_S \ [\text{m}^2/\text{m}^3]$$

berechnen.

Für den Phasenanteil $\varphi_g$ der dispersen Phase (im Falle der Hydroformylierung sind Synthesegas und organische Phase dispergiert), kann mit den Leerrohrgeschwindigkeiten der Phasen

$$\varphi_g \sim w_g/w_{gl}$$

gesetzt werden.

Die Verweilzeit $\tau$ der den Reaktor durchströmenden Phasen läßt sich angenähert nach $\tau \sim L^*\psi/W_{lg}$ berechnen. Die Verweilzeit $\tau$ beträgt bei dem erfindungsgemäßen Verfahren in der Regel weit unter einer Stunde und kann im Minutenbereich oder sogar noch darunter liegen. Dennoch werden bei dieser völlig ungewöhnlichen Fahrweise - hoher Katalysatordurchsatz im Reaktor, vergleichsweise sehr geringer Anteil an Edukt an der Reaktionsmasse, dadurch bedingt wiederum sehr kurze Verweilzeit - bei vielen Mehrphasenreaktionen überraschend hohe Raum-Zeit-Ausbeuten erzielt. Alternativ kann bei gleichen Raum-Zeit-Ausbeuten bei deutlich tieferen Temperaturen als üblich gearbeitet werden, da die Steigerung der Reaktionsgeschwindigkeit, was zum Beispiel die Minimierung von Folgereaktionen und damit verbesserte Selektivität nach sich ziehen kann, dies wirtschaftlich zuläßt.

Das erfindungsgemäße Verfahren kann sehr flexibel den unterschiedlichsten Anforderungen angepaßt werden. Für spezielle Anforderungen bieten sich folgende Ausführungsformen des erfindungsgemäßen Verfahrens an:

Erfordert der Einsatzzweck eine sehr lange Durchmischungszone oder sind Ruhezonen z. B. zur Abnahme von Stoffströmen erforderlich, so bietet sich eine kaskadierende Anordnung von Rohrreaktoren mit Einbauten oder Füllkörpern an.

Eine Kaskadierung von Rohrreaktoren oder die alternative Anordnung von gepackten und leeren Rohrabschnitten ist zu empfehlen, wenn ein besonders geringer Druckverlust gewünscht wird.

Weiterhin ist die parallele Anordnung von Rohrreaktoren oder die Verwendung eines Vielrohrreaktors, wobei die Rohre die Funktion der Einbauten übernehmen können, denkbar. Außerdem können Reaktoren mit einer Mehrfacheinspeisung von Gas über die Reaktorlänge versehen werden, wenn der Gasverbrauch so hoch ist, daß ungünstige Phasenverhältnisse von Gas zu Flüssigkeit bei der Zusammenführung der beiden Phasen vor dem Reaktor resultieren.

Die besonderen Bedingungen des erfindungsgemäßen Verfahrens lassen weitere Ausführungsformen des Verfahrens zu. So kann der hohe notwendige Kreislauf der Katalysatorphase bzw. der kontinuierlichen Phase zusätzlich zum Betrieb einer Strahldüse, die als Flüssigkeitsstrahl-Gasverdichter vor dem eigentlichen Rohrreaktor angeordnet wird, ausgenutzt werden. Dies kann zur gründlichen Vorvermischung der beiden Phasen sowie zur Verdichtung der Gasphase, was die Fahrweise bei höheren Vordrücken im Reaktor ermöglicht, eingesetzt werden. Schließlich wird, wenn man umgekehrt statt der Verdichtung des Gases die Saugwirkung ausnutzt, sogar eine Kreislaufführung von Gas unter gleichzeitiger Vorvermischung der Phasen möglich. Die von der den Katalysator enthaltende kontinuierliche Phase in den Rohrreaktor eingebrachte Energie kann so zur Dispergierung der Eduktphase bzw. mindestens eines Edukts eingesetzt werden.

Auch die Wärmeabfuhr bei stark exothermen Reaktionen wie der Hydroformylierung ist beim erfindungsgemäßen Verfahren unkritisch. Der hohe Durchsatz des Katalysatorkreislaufs wirkt als Wärmeträger, so daß selbst bei adiabatischer Fahrweise des Reaktors nur geringe Temperaturdifferenzen auftreten und eine homogene Temperaturverteilung im Reaktor ohne Temperaturspitzen resultiert. Die erzeugte Wärme läßt sich dann bequem durch einen beliebig im äußeren Katalysatorkreislauf angeordneten, konventionellen Wärmetauscher abführen oder zur Energiegewinnung ausnutzen. Zur besseren Wärmeabfuhr kann es unter Umständen günstig sein, den Katalysatorkreislauf noch höher zu fahren (also bei einem höheren B-Wert) als nach den Versuchsergebnissen notwendig ist, da über den Katalysatorkreislauf ein kleiner Temperaturgradient über den Reaktor einstellbar ist.

Das erfindungsgemäße Verfahren bietet im Vergleich zum Stand der Technik erhebliche Vorteile, genannt seien:

- Bei vergleichsweise niedrigen Temperaturen können hohe Raum-Zeit-Ausbeuten erreicht werden.
- Die Bildung von Nebenprodukten ist extrem niedrig, Werte von 1 - 2 Gew.-% und darunter sind möglich.
- Der Katalysator wird geschont, die Desaktivierung ist sehr gering, eine kontinuierliche Ausschleusung entfällt.

Im Falle der Herstellung von Aldehyden durch Hydroformylierung von Olefinen mit dem erfindungsgemäßen Verfahren kommen weitere Vorteile hinzu:

- Aufgrund der höheren Reaktionsgeschwindigkeit kann dieses Verfahren auch für die Hydroformylierung von höheren Olefinen mit mehr als 10 Kohlenstoffatomen wirtschaftlich genutzt werden.
- Bei gasförmigen Olefinen kann der nach einem Teilumsatz verbleibende Eduktanteil durch einfache Rückführung mittels einer Strahldüse zurückgeführt werden.

Im Folgenden wird das erfindungsgemäße Verfahren zur Hydroformylierung von Olefinen mit Synthesegas in einem Mehrphasensystem näher beschrieben. Als kontinuierliche Phase wird die Katalysatorphase eingesetzt und mittels einer Pumpe durch einen Rohrreaktor gepumpt.

Als Lösungsmittel für die Herstellung der Katalysatorlösung bzw. -phase sind alle diejenigen Solventien geeignet, die folgende Bedingungen erfüllen:

- Das Lösungsmittel ist in der Produktphase wenig löslich.
- Das Produkt löst sich nur wenig in der Katalysatorphase, die aus Katalysator und Lösungsmittel besteht.
- Das Lösungsmittel hat für den eingesetzten Katalysator eine genügend hohe Löslichkeit.

Das Lösungsmittel kann als Zusatz Phasentransfer-, oberflächenaktive- oder amphiphile Reagenzien oder Tenside enthalten. Als bevorzugtes Lösungsmittel wird Wasser eingesetzt.

Als Hydroformylierungskatalysatoren können die Metalle der 8. Nebengruppe (Fe, Co, Ni, Ru, Rh, Pd, Os, Ir, Pt) eingesetzt werden. Diese Metalle bzw. Verbindungen dieser Metalle sollten unter den Reaktionsbedingungen in der Katalysatorphase, aber nicht in der Produktphase löslich sein. Werden wäßrige Katalysatorlösungen eingesetzt, erfordert dies wasserlösliche Metallverbindungen. Als bevorzugter Katalysator werden Rhodium bzw. wasserlösliche Rhodiumverbindungen eingesetzt. Geeignete Rhodiumsalze sind z. B. Rhodium-III-sulfat, Rhodium-III-nitrat, Rhodium-III-carboxylate wie Rhodiumacetat, Rhodiumpropionat, Rhodiumbutyrat oder Rhodium-2-ethylhexanoat.

Die Art der Liganden hängt vom verwendeten Metall und Solvens der Katalysatorlösung ab. Für diese Komplexe gilt, daß sie katalytisch wirksam sind und ihre katalytische Wirkung im Dauerbetrieb nicht verlieren. Bedingung dafür ist, daß sich die Liganden nicht verändern, wie z.B. durch Reaktion mit dem Lösungsmittel.

Als Liganden für die katalytisch aktiven Metalle können Triarylphosphine eingesetzt werden. Geeignete Phosphine weisen ein oder zwei Phosphoratome auf, die je Phosphoratom drei Arylreste besitzen, wobei die Arylreste gleich oder verschieden sind und für einen Phenyl-, Naphthyl-, Biphenyl-, Phenylnaphtyl- oder Binaphtyl-Rest, insbesondere Phenyl-, Biphenyl- oder Binaphtyl-Rest stehen. Die Arylreste können mit dem Phosphoratom direkt oder über eine -$(CH_2)_x$-Gruppe, worin x für eine ganze Zahl von 1 bis 4, bevorzugt 1 bis 2, besonders bevorzugt 1 steht, verbunden sein. Für wasserlösliche Katalysatorsysteme sollte ein Ligand vorzugsweise drei -$(SO_3)M$ Reste enthalten, worin M gleich oder verschieden ist und für H, ein Alkalimetallion, ein Ammoniumion, ein quarternäres Ammoniumion, ein (rechnerisch halbes) Erdalkalimetallion oder Zinkion steht.

Die -$SO_3M$ Reste sind üblicherweise Substituenten an den Arylresten und verleihen den Triarylphosphinen die erforderliche Wasserlöslichkeit. Ein bevorzugtes sulfoniertes Triarylphosphin mit einem Phosphoratom ist das Trinatrium-tri-(m-sulfophenyl)phosphin.

Neben den Sulfonato-Einheiten (-$SO_3M$) können auch andere polare Gruppen, wie z.B. Carboxylato-Einheiten eingesetzt werden.

Man kann die wäßrige Phase unmittelbar in die Hydroformylierung einsetzen oder sie zuvor einer Präformierung des Katalysators unter Reaktionsbedingungen unterwerfen, um sie anschließend in präformierter Form zu verwenden. Die wäßrige Katalysatorlösung läßt sich jedoch auch auf vergleichsweise einfache Art herstellen, indem man ein wasserlösliches Metallsalz und die wasserlöslichen Liganden in Wasser löst und zur Komplexbildung bringt.

Die im erfindungsgemäßen Verfahren nötige Metallsalzkonzentration kann über einen weiten Bereich eingestellt werden. Der höchste Wert ist durch die Löslichkeit vorgegeben. Die Reaktionsgeschwindigkeit hängt auch von der Metallsalzkonzentration ab. In der Regel werden bei höheren Metallsalzkonzentrationen höhere Reaktionsgeschwindigkeiten erreicht. Andererseits bedeuten höhere Metallsalzkonzentrationen höhere Kosten. Es kann daher je nach Reaktivität des Edukts und der sonstigen Reaktionsbedingungen ein Optimum gewählt werden. Der Rhodiumgehalt in der Katalysatorphase beträgt üblicherweise 20 ppm bis 2000 ppm, bevorzugt 100 bis 1000 ppm.

Im eingesetzten Katalysatorsystem kann das molare Verhältnis zwischen Metall und Liganden variiert werden, um das Optimum für jede einzelne Umsetzung zu erreichen. Dieses Verhältnis liegt zwischen 1/5 und 1/200, insbesondere zwischen 1/10 und 1/60.

Der pH-Wert der Katalysatorlösung kann für die Hydroformylierung jedes Olefins hinsichtlich der Selektivität der Aldehydbildung optimiert werden. Er liegt zwischen 2 und 8, vorzugsweise zwischen 3,0 und 5,5.

Als Edukte zur Hydroformylierung können olefinische Verbindungen mit 2-25 Kohlenstoffatomen, bevorzugt mit 2-12 Kohlenstoffatomen eingesetzt werden. Die olefinischen Verbindungen können eine oder mehrere Kohlenstoff-Kohlenstoff-Doppelbindung enthalten, die jeweils endständig oder innenständig angeordnet sein können. Bevorzugt sind olefinische Verbindungen mit endständiger Kohlenstoff-Kohlenstoff-Doppelbindung. Dabei kann ein Olefin einheitlicher Struktur eingesemtzt werden. Es können auch Olefingemische eingesetzt werden. Das Gemisch kann aus isomeren Olefinen gleicher Anzahl von Kohlenstoffatomen oder aus Olefinen unterschiedlicher Anzahl von Kohlenstoffatomen oder aus einem Gemisch, das sowohl isomere Olefine als auch Olefine mit unterschiedlicher Anzahl von Kohlenstoffatomen enthält, bestehen. Weiterhin können die Olefine oder Olefingemische unter Reaktionsbedingungen inerte Stoffe wie aliphatische Kohlenwasserstoffe enthalten.

Im erfindungsgemäßen Verfahren können Olefine aus den verschiedensten Quellen eingesetzt werden. Beispielsweise seien Olefine aus Crackprozessen, Dehydrierungen oder aus der Fischer-Tropsch-Synthese genannt. Ebenso sind Olefine oder Olefingemische, die durch Dimerisierung, Oligomerisierung, Codimerisierung, Cooligomerisierung oder Metathese von Olefinen entstanden sind, geeignete Edukte. Die eingesetzten Olefine können (unter Normalbedingungen) gasförmig, flüssig oder fest sein. Feste Olefine werden als Lösungen eingesetzt. Als Lösungsmittel werden inerte, in der Katalysatorphase kaum lösliche Flüssigkeiten verwendet. Besonders bevorzugt sind Lösungsmittel, die einen höheren Siedepunkt als die herzustellenden Produkte haben, da hierdurch die destillative Abtrennung und Rückführung erleichtert wird.

Bevorzugt werden im erfindungsgemäßen Verfahren α-olefinische Verbindungen eingesetzt. Beispiele für geeignete α-olefinische Verbindungen sind 1-Alkene, Alkylalkanoate, Alkylenalkanoate, Alkenylalkylether und Alkenole, wie z. B. Propen, Buten, Penten, Butadien, Pentadien, 1-Hexen, 1-Hepten, 1-Octen, 1-Nonen, 1-Decen, 1-Undecen, 1-Dodecen, 1-Hexadecen, 2-Ethyl-1-hexen, 1,4-Hexadien, 1,7-Octadien, 3-Cyclohexyl-1-buten, Styrol, 4-Vinylcyclohexen, Allylacetat, Vinylformiat, Vinylacetat, Vinylpropionat, Allylmethylether, Vinylmethylether, Vinylethylether, Allylalkohol, 3-Phenyl-1-propen, Hex-1-en-4-ol, Oct-1-en-4-ol, 3-Butenylacetat, Allylpropionat, Allylbutyrat, n-Propyl-7-octenoat, 7-Octensäure, 5-Hexenamid, 1-Methoxy-2,7-octadien, 3-Methoxy-1,7-octadien insbesondere Propen, 1-Buten, technisch verfügbare Gemische, die im wesentlichen 1-Buten, 2-Buten und i-Buten enthalten, und/oder 1-Penten.

Produkte der Hydroformylierung von Olefinen sind um ein Kohlenstoffatom längere Aldehyde und ggf. Alkohole. Die nach dem erfindungsgemäßen Verfahren hergestellten Aldehyde können zur Herstellung von Alkoholen durch Hydrierung verwendet werden. Die so erhaltenen Alkohole sind wiederum Vorstufen für Weichmacher, z. B. als Phthalsäurediester oder Detergenzien.

Weiterhin finden die Aldehyde nach dem Verfahren der Erfindung Verwendung in Aldolkondensationen und bei der Herstellung von Carbonsäuren durch Oxidation.

Als Hydroformylierungsagens werden Gemische aus Wasserstoff und Kohlenmonoxid (Synthesegas) oder weitere Gemische aus Wasserstoff, Kohlenmonoxid und unter Reaktionsbedingungen inerten Stoffen eingesetzt.

Bei Einsatz von flüssigen Olefinen oder festen Olefinen in Lösung ist es günstig, das Hydroformylierungsagens im Überschuß einzusetzen, damit ein möglichst vollständiger Umsatz erreicht wird. Dies senkt den Aufwand für die Aufarbeitung. Bei Einsatz von gasförmigen Olefinen kann es dagegen günstig sein, daß Hydroformylierungsreagens im Unterschuß zu verwenden, da das überschüssige gasförmige Olefin sich vom flüssigen Produkt trennt und wieder in den Prozess zurückgeführt werden kann.

Das molare Verhältnis von Olefin zu Wasserstoff und Olefin zu Kohlenmonoxid kann größer, kleiner oder gleich 1 sein.

Das erfindungsgemäße Verfahren der Hydroformylierung ist bei Einsatz eines gasförmigen Olefins zunächst eine Zweiphasenreaktion, wobei sich während der Reaktion eine flüssige Produktphase bildet und somit ein Dreiphasensystem entsteht. Bei Einsatz eines flüssigen Olefins liegt von Anfang an ein Dreiphasensystem vor.

Das erfindungsgemäße Verfahren kann in einen oder mehreren Rohrreaktoren mit Einbauten entsprechend der vorangegangenen Beschreibung ausgeführt werden.

Im erfindungsgemäßen Verfahren ist die Katalysatorphase die kontinuierliche Phase; ein Massenverhältnis zwischen der Katalysatorphase und der oder den dispersen Phasen im Bereich von 2/1 bis 3500/1, bevorzugt im Bereich 40/1 bis 2500/1 ist daher zweckmäßig.

Im erfindungsgemäßen Verfahren zur Hydroformylierung von Olefinen, kann das Massenverhältnis zwischen Katalysatorphase und olefinischer Phase am Reaktoreingang im Bereich 5000/1 bis 4/1, vorzugsweise im Bereich 2000/1 bis 50/1 liegen. Das Massenverhältnis zwischen Katalysatorphase und dem Hydroformylierungsagens (in der Regel Synthesegas) beträgt 4/1 bis 10000/1, vorzugsweise 200/1 bis 4000/1.

Die Reaktanden können vorgewärmt, d. h. im Bereich der Reaktionstemperatur, oder kalt zugeführt werden. Aufgrund des hohen Phasenverhältnisses mit der Katalysatorphase kann die Vorwärmung auch durch die Prozeßwärme erfolgen.

Bei dem erfindungsgemäßen Verfahren zur Hydroformylierung von Olefinen erfolgt die Umsetzung in einem Temperaturbereich von 20 °C bis 250 °C, vorzugsweise im Bereich von 90 °C bis 150 °C; hierbei liegt der Gesamtdruck zwischen 10 bar und 300 bar, vorzugsweise zwischen 20 bar und 150 bar.

Das Reaktionsrohr kann im Gleichstrom von oben nach unten oder umgekehrt durchströmt werden. Aus Sicherheitsgründen wird der Beschickung von oben der Vorzug gegeben.

Die Reaktionswärme kann über verschiedene Wärmeaustauscher abgeführt werden. Die Wärmeaustauscher müssen hierbei nicht in der Nähe des Reaktionsraums sein, sondern können auch beliebig außerhalb des Reaktors liegen. Die einzelnen Wärmeflüsse sind abhängig von der spezifischen Reaktionswärme sowie von den gewünschten Temperaturen im Reaktor und in den Aufarbeitungsvorrichtungen.

Die abgeführte Reaktionswärme kann so sehr einfach genutzt werden, z.B. im Prozess selbst, zur Beheizung einer Destillationseinrichtung oder zur Erzeugung von Dampf.

Das den Reaktor verlassende Gemisch kann für den Fall des Einsatzes von gasförmigen Olefinen oder bei einem unvollständigen Umsatz in einem Gas-Flüssig-Trennbehälter entgast werden. Die Gas-Flüssigkeit-Trennung kann beim gleichen Druck, der am Reaktorausgang herrscht, erfolgen. Dies ist besonders dann von Vorteil, wenn zumindestens ein Teil des Entspannungsgas in den Reaktor zurückgeführt wird. Ansonsten kann auch bei tieferem Druck (bis hinunter auf 1 bar) entspannt werden.

Der abgetrennte Gasstrom kann vollständig oder teilweise in den Reaktor zurückgeführt werden.

Diese Rückführung kann auf bekannte Weise, z.B. durch eine Strahl- oder Mischdüse, die im Katalysatorkreislaufstrom vor dem Reaktor angebracht ist, oder durch einen Kreisgasverdichter, erreicht werden. Aus energetischen Überlegungen wird bevorzugt eine Strahl- oder Mischdüse, die im Katalysatorkreislaufstrom vor dem Reaktor angebracht ist, verwendet.

Die restliche oder wahlweise die gesamte Gasmenge kann gekühlt oder ungekühlt in ein Abgasverwertungssystem eingeleitet werden. Bei Verwendung eines Kühlers kann das im Kühler anfallende Gaskondensat über eine Leitung in den Gas-Flüssig-Trennbehälter geleitet werden.

Das entgaste Flüssigkeitsgemisch wird in einem Flüssig-Flüssig-Trennbehälter in die Katalysatorphase und in die Produktphase mechanisch getrennt. Dies kann in Absitzbehältern verschiedener Bauart oder Zentrifugen erfolgen. Aus Kostengründen werden Absitzbehälter bevorzugt.

Die Verweilzeiten in der Trennvorrichtung sind zwar nicht grundsätzlich kritisch, werden aber vorteilhaft klein gehalten. Dies hat folgende Vorteile: Die Trennvorrichtung ist klein und das Investment dafür entsprechend gering. Bei kurzen Verweilzeiten treten praktisch keine Nebenreaktionen im Trennbehälter auf. Damit die Trennung der Phasen schnell erfolgt, müssen die Dichteunterschiede der beiden Phasen genügend groß und ihre Viskositäten gering sein. Alle vier Größen sind eine Funktion der Temperatur und können durch orientierende Versuche leicht ermittelt werden.

Darüber hinaus kann die Dichte und Viskosität der Katalysatorlösung durch Auswahl des Solvens und der Katalysatorkonzentration variiert werden. Als weitere Möglichkeit kann die Dichte und Viskosität der Produktphase durch Zusatz eines Lösungsmittel verändert werden.

Die Phasentrennung kann in einem weiten Temperaturbereich vorgenommen werden. Dabei kann die Trenntemperatur auch höher sein als die Temperatur des Reaktionsaustrags am Reaktorausgang. Aus energetischen Gründen ist es jedoch nicht günstig, eine höhere Temperatur als die Flüssigkeitstemperatur im Gasabscheider anzuwenden. Als tiefstmögliche Temperatur ist der Stockpunkt einer der beiden flüssigen Phasen anzusehen. Im Hinblick auf kurze Trennzeiten werden jedoch, wie oben erwähnt, keine zu tiefen Temperaturen gewählt.

Der Produktstrom wird nach bekannten Verfahren aufgetrennt, z.B. durch Destillation.

Die abgetrennte Katalysatorlösung wird, ggf. nach Ausschleusung einer kleinen Teilmenge und entsprechendem Ersatz durch frische Katalysatorlösung, in den Reaktor zurückgeführt.

Die folgenden Beispiele sollen die Erfindung beschreiben, ohne deren Anwendungsbreite einzuschränken, die aus den Patentansprüchen hervorgeht.

## Hydroformylierung von Olefinen

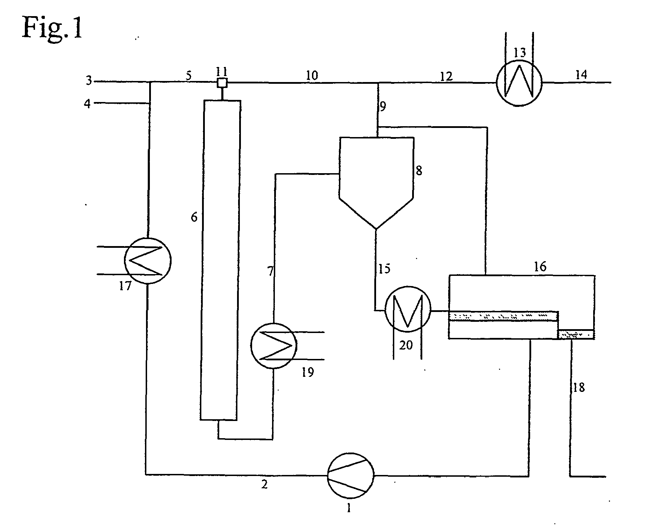

Die Hydroformylierung von Olefinen erfolgte in einer Versuchsapparatur, die schematisch in Fig. 1 dargestellt ist. Hierin wird mit einer Pumpe 1 der wäßrige Katalysator im Kreislauf gepumpt. Zur Katalysatorphase 2 wird Olefin 3 und Synthesegas 4 zugemischt. Die Mehrphasenmischung 5 wird durch den Rohrreaktor 6 gepumpt, der mit statischen Mischelementen versehen ist. Die resultierende Mischung 7, bestehend aus Produkt, nicht umgesetztem Edukt und dem Katalysator werden im Gasabscheider 8 entgast. Das Gas 9, bestehend aus Synthesegas, gegebenenfalls gasförmigen Olefin und angereicherten Inerten wird zum größten Teil dem Reaktor 6 über eine Gasrückführungsleitung 10 mit Hilfe einer Mischdüse bzw. Strahldüse 11 erneut zugeführt. Ein kleiner Teil des Gasstroms 9 wird über Leitung 12 ausgeschleust. Durch geeignete Kühlung 13 und Rücklauf können Olefinverluste minimiert werden. Die Ausschleusung 14 reduziert sich auf angereicherte Inerte und kleine Mengen nicht umgesetzten Synthesegases.

Der nach der Entgasung 8 anfallende Flüssigkeitsstrom 15 wird in einen Phasentrennbehälter 16 geleitet. Hier wird die wäßrige Katalysatorphase 2 abgetrennt und erneut dem Kreislauf zugeführt. Die Reaktionswärme kann über außerhalb des Reaktors liegende Wärmetauscher 17, 19, 20 abgeführt werden. Das Produkt wird über Leitung 18 entnommen und ggf. weiter gereinigt.

Die Beispiele 1-7 beschreiben die Hydroformylierung von Propen durch das erfindungsgemäße Verfahren.

Beispiele 1-5:

Es wurde ein Reaktor 6 mit einer Länge von 3 m und einem Durchmesser von 17,3 mm eingesetzt, der statische Mischelemente der Fa. Sulzer mit einem hydraulischen Durchmesser von 2 mm enthielt.

Für dieses Beispiel war die Leitung 10 zur Rückführung des Gases geschlossen. Als Lösungsmittel für den Katalysator wurde Wasser eingesetzt. Der pH-Wert betrug 7. Der Reaktor wurde mit einer Katalysatorbelastung von 400 kg/h bei einer Temperatur von 120 °C durchströmt. Der Reaktionsdruck betrug 50 bar. Als Katalysator wurde Rhodium mit einer Konzentration von 800 ppm, bezogen auf die Lösungsmittelphase eingesetzt.. Als Ligand wurde Tri(m-sulfophenyl) phosphin (TSTPP) in Form seines Natriumsalzes eingesetzt, das Verhältnis P/Rh betrug 60. Für den Versuch 2 wurden die Reaktionsbedingungen von Versuch 1 eingestellt, mit der Abweichung, daß die Reaktionstemperatur 130 °C betrug. Für den Versuch 3 wurden die Reaktionsbedingungen von Versuch 1 eingestellt, mit der Abweichung, daß der Reaktionsdruck 70 bar betrug. Für den Versuch 4 wurden die Reaktionsbedingungen von Beispiel 1 eingestellt, mit der Abweichung, daß der pH-Wert der Katalysatorlösung auf 4 eingestellt wurde. Für den Versuch 5 wurden die Reaktionsbedingungen von Beispiel 1 eingestellt, mit der Abweichung, daß die Katalysatorbelastung des Reaktors 300 kg/h betrug. Die zugefahrenen Stoffmengenströme der Edukte sowie der Produkte sind in der Tabelle in mol/h angegeben.

| Beispiel | 1 | 2 | 3 | 4 | 5 |
|---|---|---|---|---|---|
| RZA [t/(m$^3$*h)] | 0,98 | 1,39 | 1,23 | 1,00 | 0,71 |
| B | 13,16 | 13,34 | 13,16 | 13,16 | 8,06 |
| Edukt | | | | | |
| CO | 22,79 | 16,99 | 28,24 | 23,92 | 12,73 |
| $H_2$ | 21,28 | 15,87 | 26,38 | 22,87 | 11,89 |
| $N_2$ | 0,12 | 0,09 | 0,15 | 0,10 | 0,07 |
| Propen | 51,49 | 35,57 | 31,13 | 36,01 | 33,45 |
| Propan | 0,17 | 0,12 | 0,10 | 0,11 | 0,11 |
| Produkt | | | | | |
| CO | 2,15 | 2,09 | 2,08 | 0,66 | 2,11 |
| $H_2$ | 0,89 | 0,93 | 0,98 | 0,37 | 0,97 |
| Propen | 3,35 | 6,73 | 3,04 | 3,45 | 4,00 |
| i-Butanal | 0,24 | 0,51 | 0,53 | 0,39 | 0,20 |
| n-Butanal | 5,46 | 11,03 | 9,82 | 6,19 | 4,66 |
| i-Butanol | <0,01 | <0,01 | <0,01 | <0,01 | <0,01 |
| n-Butanol | 0,07 | 0,07 | 0,07 | 0,06 | 0,07 |
| 2-Ethylhexanal | 0,09 | 0,03 | 0,08 | <0,01 | 0,02 |
| 2-Ethylhexenal | 0,26 | 0,19 | 0,18 | <0,01 | 0,09 |
| Abgas | | | | | |
| CO | 9,50 | 3,06 | 13,23 | 13,72 | 6,81 |
| $H_2$ | 8,45 | 1,46 | 9,97 | 10,82 | 3,59 |
| $N_2$ | 0,29 | 0,10 | 0,09 | 0,10 | 0,16 |
| Propen | 37,37 | 13,56 | 17,72 | 19,47 | 36,93 |
| Propan | 0,17 | 0,10 | 0,17 | 0,10 | 0,09 |
| i-Butanal | 0,24 | 0,16 | 0,15 | 0,18 | 0,14 |
| n-Butanal | 4,59 | 3,13 | 2,72 | 2,62 | 2,56 |

Beispiele 6 und 7:

Diese Beispiele illustrieren den Einsatz der Gasrückführungsleitung 10. Hierdurch läßt sich der Verlust an Wertstoffen über die Abgasleitung minimieren. Bereits bei diesen Beispielen sind Umsätze >90% leicht zu erreichen. Aufgrund des Kühlers 13 kann die Ausschleusung von Propen im Abgas nahezu vollständig verhindert werden, so daß Propen nur noch mit dem Flüssigprodukt austritt. Das aus dem Phasentrennbehälter abgezogene Produkt 18, das noch Propen enthält wird dem Stand der Technik entsprechend aufgearbeitet, indem man es z.B. mit dem Synthesegas austreibt oder destillativ abtrennt. Hierdurch sind Propenumsätze von >99 % ohne Einbußen in der Raum-Zeit-Ausbeute möglich.

In den Beispielen 6 und 7 wurde der Reaktor mit einer Katalysatorbelastung von 400 kg/h bei einer Temperatur von 120°C durchströmt. Der Reaktionsdruck betrug 50 bar. Die Konzentration des Rhodiums betrug 800 ppm bezogen auf die Lösungsmittelphase. Als Ligand wurde TSTPP in Form seines Natriumsalzes eingesetzt, das Verhältnis P/Rh betrug 60. Die zugefahrenen Stoffmengenströme der Edukte sowie der Produkte sind in der Tabelle in mol/h angegeben.

| Beispiel | 6 | 7 |
|---|---|---|
| RZA[t/(m$^3$*h)] | 1,06 | 1,04 |
| B | 12,12 | 12,12 |
| Propen-Umsatz | 93,70% | 92,33% |
| Synthesegas-Umsatz | 92,09% | 90,27% |
| Eingang | | |
| Edukt | | |
| CO | 10,41 | 10,23 |
| H$_2$ | 9,56 | 9,63 |
| N$_2$ | 0,07 | 0,07 |
| Propen | 12,42 | 12,43 |
| Propan | 0,07 | 0,07 |
| Produkt | | |
| CO | 1,27 | 1,65 |
| H$_2$ | 0,24 | 0,39 |
| Propen | 0,58 | 0,73 |
| i-Butanal | 0,86 | 0,77 |
| n-Butanal | 8,04 | 8,24 |
| i-Butanol | <0,001 | <0,001 |
| n-Butanol | 0,13 | 0,11 |
| 2-Ethylhexanal | 0,15 | 0,12 |
| 2-Ethylhexenal | 0,29 | 0,18 |
| Abgas | | |
| CO | 0,18 | 0,09 |
| H$_2$ | 0,09 | 0,05 |
| N$_2$ | 0,01 | 0,01 |
| Propen | 0,10 | 0,09 |
| Propan | 0,003 | 0,002 |

Hydroformylierung von Hexen-1

Beispiel 8:

Dieses Beispiel beschreibt den Einsatz des erfindungsgemäßen Verfahrens zur kontinuierlichen Hydroformylierung von 1-Hexen. Der Umsatzverlauf über einen längeren Reaktor wurde ermittelt, indem das erhaltene Rohprodukt immer wieder neu eingesetzt wurde. Man erhält somit Werte, wie aus einem mehrere Meter langen Reaktor, bei dem jeweils nach 1 m Gas neu eindosiert wird. Als Lösungsmittel für den Rhodium-Katalysator wurde Wasser eingesetzt.

EP 1 057 524 B1

Der Reaktor mit einem Volumen von 235 ml (1m Länge) wurde mit einer Katalysatorbelastung von 400 kg/h bei einer Temperatur von 130°C durchströmt. Der Reaktionsdruck betrug 30 bar. Die Konzentration des Rhodiums betrug 800 ppm bezogen auf die Lösungsmittelphase. Als Ligand wurde TSTPP in Form seines Natriumsalzes eingesetzt, das Verhältnis P/Rh betrug 60. Die Synthesegasbelastung betrug im Mittel 132,8 Nl/h. Die Eingangsströmung des flüssigen Eduktes wurde auf 1,3 l/h eingestellt. Nach 8 Durchgängen (= 8m Reaktorlänge) werden knapp 90% Umsatz erreicht, die RZA liegt aber immer noch bei 0,44 t/(m³*h) über die gesamte Reaktorlänge gerechnet.

Dieser Wert liegt weit über technisch bekannten Daten. Die Gesamtselektivität nach 8 Durchgängen betrug fast 93%.

| Anzahl Durchgänge | 1 | 2 | 3 | 4 | 5 | 6 | 7 | 8 |
|---|---|---|---|---|---|---|---|---|
| Gesamt-Umsatz [%] | 19,1 | 36,9 | 49,8 | 59,8 | 68,5 | 76,5 | 82,9 | 86,8 |
| Hochsieder | 0 | 0,37 | 0,28 | 2,1 | 2,65 | 4,29 | 4,59 | 4,62 |
| Gesamt-Selektivität [%] | 85,1 | 89,1 | 91,7 | 92,1 | 92,4 | 92,8 | 93,1 | 92,9 |
| Gesamt-RZA[t/(m³*h)] | 0,748 | 0,743 | 0,689 | 0,619 | 0,568 | 0,521 | 0,486 | 0,444 |
| B | 14,26 | 14,26 | 14,26 | 14,26 | 14,26 | 14,26 | 14,26 | 14,26 |

Beispiel 9:

Hydroformylierung von 1-Decen

Es wurde ein Reaktor mit einer Länge von 6 m und einem Durchmesser von 17,3 mm eingesetzt, der statische Mischelemente der Fa. Sulzer mit einem hydraulischen Durchmesser von 2 mm enthielt.

Für dieses Beispiel war die Leitung 10 zur Rückführung des Gases geschlossen. Als Lösungsmittel für den Katalysator wurde Wasser eingesetzt. Der pH-Wert betrug 4,5. Der Reaktor wurde mit einer Katalysatorbelastung von 400 kg/h bei einer Temperatur von 125 °C durchströmt. Der Reaktionsdruck betrug 70 bar. Die Konzentration des Rhodiums betrug 800 ppm bezogen auf die Lösungsmittelphase. Als Ligand wurde TSTPP in Form seines Natriumsalzes eingesetzt, das Verhältnis P/Rh betrug 5. Die zugefahrenen Stoffmengenströme der Edukte sowie der Produkte sind in der Tabelle in mol/h angegeben.

| Beispiel | 9 |
|---|---|
| RZA [t/(m³*h)] | 0,05 |
| B | 13,96 |
| Edukt | |
| CO | 11,38 |
| $H_2$ | 10,52 |
| $N_2$ | 0,05 |
| 1-Decen | 7,12 |
| Produkt | |
| 1-Decen | 6,67 |
| Undecanal | 0,34 |
| 2-Methyldecanal | 0,11 |
| Abgas | |
| CO | 10,36 |
| $H_2$ | 9,89 |
| $N_2$ | 0,05 |

14

**Patentansprüche**

1. Verfahren zur katalytischen Durchführung von Mehrphasenreaktionen in einem Rohrreaktor **dadurch gekennzeichnet,**
   **daß** der Katalysator in der kontinuierlichen Phase und mindestens ein Edukt in einer dispergierten Phase enthalten ist und der Belastungsfaktor B des Rohrreaktors gleich oder größer 0,8 ist und daß durch die Mehrphasenreaktion Olefine hydroformyliert werden.

2. Verfahren nach Anspruch 1,
   **dadurch gekennzeichnet,**
   **daß** die Olefine 2 bis 25 Kohlenstoffatome enthalten.

3. Verfahren nach Anspruch 1 oder 2,
   **dadurch gekennzeichnet,**
   **daß** als Katalysator ein Metall der 8. Nebengruppe eingesetzt wird.

4. Verfahren nach einem der Ansprüche 1 bis 3,
   **dadurch gekennzeichnet,**
   **daß** als Katalysator Rhodium eingesetzt wird.

5. Verfahren nach einem der Ansprüch 1 bis 4,
   **dadurch gekennzeichnet,**
   **daß** als Katalysator wasserlösliche Rhodiumverbindungen eingesetzt werden.

6. Verfahren nach einem der Ansprüche 1 bis 5,
   **dadurch gekennzeichnet,**
   **daß** als kontinuierliche Phase Wasser oder ein Gemisch aus Wasser und einem organischen Lösungsmittel eingesetzt wird.

7. Verfahren nach einem der Ansprüche 1 bis 6,
   **dadurch gekennzeichnet,**
   **daß** der Belastungsfaktor B größer oder gleich 0,9 ist.

8. Verfahren nach einem der Ansprüche 1 bis 6,
   **dadurch gekennzeichnet,**
   **daß** der Belastungsfaktor B größer oder gleich 1,0 ist.

9. Verfahren nach einem der Ansprüche 1 bis 8,
   **dadurch gekennzeichnet,**
   **daß** das Massenverhältnis der kontinuierlichen Phase zu der oder den dispersen Phasen größer 2 ist.

10. Verfahren nach einem der Ansprüche 1 bis 9,
    **dadurch gekennzeichnet,**
    **daß** die kontinuierliche Phase vor dem Rohrreaktor eine Strahldüse antreibt.

11. Verfahren nach einem der Ansprüche 1 bis 10,
    **dadurch gekennzeichnet,**
    **daß** mindestens ein Edukt durch die von der kontinuierlichen Phase in den Rohrreaktor eingebrachte Energie dispergiert wird.

**Claims**

1. Process for carrying out multiphase reactions catalytically in a tubular reactor **characterized in that** the catalyst is present in the continuous phase and at least one starting material in a disperse phase and the loading factor B of the tubular reactor is equal to or greater than 0.8, and **in that** olefins are hydroformylated via the multiphase reaction.

2. Process according to Claim 1, **characterized in that** the olefins contain 2 to 25 carbon atoms.

**3.** Process according to Claim 1 or 2, **characterized in that** the catalyst used is a metal of subgroup 8.

**4.** Process according to one of Claims 1 to 3, **characterized in that** the catalyst used is rhodium.

**5.** Process according to one of Claims 1 to 4, **characterized in that** the catalyst used is a water-soluble rhodium compound.

**6.** Process according to one of Claims 1 to 5, **characterized in that** the continuous phase used is water or a mixture of water and an organic solvent.

**7.** Process according to one of Claims 1 to 6, **characterized in that** the loading factor B is greater than or equal to 0.9.

**8.** Process according to one of Claims 1 to 6, **characterized in that** the loading factor B is greater than or equal to 1.0.

**9.** Process according to one of Claims 1 to 8, **characterized in that** the mass ratio of the continuous phase to the disperse phase or phases is greater than 2.

**10.** Process according to one of Claims 1 to 9, **characterized in that** the continuous phase drives a jet nozzle upstream of the tubular reactor.

**11.** Process according to one of Claims 1 to 10, **characterized in that** at least one starting material is dispersed by the energy introduced into the tubular reactor by the continuous phase.


**Revendications**

**1.** Procédé pour la réalisation catalytique de réactions à plusieurs phases dans un réacteur tubulaire, **caractérisé en ce que** le catalyseur est contenu dans la phase continue et au moins un produit de départ est contenu dans une phase dispersée et le facteur de charge B du réacteur tubulaire est égal ou supérieur à 0,8 et **en ce que** la réaction à plusieurs phases sert à hydroformyler les oléfines.

**2.** Procédé selon la revendication 1, **caractérisé en ce que** les oléfines contiennent 2 à 25 atomes de carbone.

**3.** Procédé selon la revendication 1 ou 2, **caractérisé en ce qu'**on utilise comme catalyseur un métal du 8ème groupe secondaire.

**4.** Procédé selon l'une quelconque des revendications 1 à 3, **caractérisé en ce qu'**on utilise, comme catalyseur, du rhodium.

**5.** Procédé selon l'une quelconque des revendications 1 à 4, **caractérisé en ce qu'**on utilise, comme catalyseur, des composés du rhodium solubles dans l'eau.

**6.** Procédé selon l'une quelconque des revendications 1 à 5, **caractérisé en ce qu'**on utilise comme phase continue de l'eau ou un mélange d'eau et d'un solvant organique.

**7.** Procédé selon l'une quelconque des revendications 1 à 6, **caractérisé en ce que** le facteur de charge B est supérieur ou égal à 0,9.

**8.** Procédé selon l'une quelconque des revendications 1 à 6, **caractérisé en ce que** le facteur de charge B est supérieur ou égal à 1,0.

**9.** Procédé selon l'une quelconque des revendications 1 à 8, **caractérisé en ce que** le rapport massique de la phase continue à la ou aux phases dispersées est supérieur à 2.

**10.** Procédé selon l'une quelconque des revendications 1 à 9, **caractérisé en ce que** la phase continue entraîne un pulvérisateur en amont du réacteur tubulaire.

**11.** Procédé selon l'une quelconque des revendications 1 à 10, **caractérisé en ce qu'**au moins un produit de départ est dispersé par l'énergie introduite par la phase continue dans le réacteur tubulaire.

# Fig.1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3234701

- DE 2715685

**In der Beschreibung aufgeführte Nicht-Patentliteratur**

- **B.CORNILS ; W.HERRMANN.** Aqueous-Phase Organometallic Catalysis, Concepts and Application. Verlag Wiley-VCH, 308-310
- **Y. SATO ; T.HIROSE ; F. TAKAHASHI ; M. TODA.** Pressure Loss and Liquid Hold Up in Packed Bed Reactor with Cocurrent Gas-Liquid Down Flow. *J. Chem. Eng. Of Japan,* 1973, vol. 6 (2), 147-152
- **D. SWEENEY.** A Correlation for Pressure Drop in Two-Phase Concurrent Flow in Packed Beds. *AlChE-Journal,* Juli 1967, 663-669

- **V. W. WEEKMAN ; J. E. MYERS.** Fluid-Flow Characteristics of Concurrent Gas-Liquid Flow in Packed Beds. *AIChE-Journal,* November 1964, vol. 10 (6), 951-957
- **R. P. LARKINS ; R. P. WHITE ; D. W. JEFFREY.** Two-Phase Concurrent Flow in Packed Beds. *AlChE-Journal,* Juni 1961, vol. 7 (2), 231-239
- **N. MIDOUX ; M. FAVIER ; J.- C. CHARPENTIER.** Flow Pattern, Pressure Loss and Liquid Holdup Data in Gas-Liquid Down-Flow Packed Beds with Foaming and Nonfoaming Liquids. *J. Chem. Eng. Of Japan,* 1976, vol. 9 (5), 350-356